# EUROPEAN PATENT APPLICATION

(11) **EP 2 614 890 A1**
(43) Date of publication of application: **17.07.2013**
(21) Application number: 12150609.1
(22) Date of filing: 10.01.2012
(51) Int. Cl.: B01J 39/04, B09B 3/00, C02F 1/42, C02F 11/04

(54) **A method for operating a biogas plant**

(71) Applicant: RE-N Technology ApS, 4320 Lejre (DK)
(72) Inventor: Wennergren, Bo, 22592 Lund (SE); Christensen, Jens Tradsborg, 11826 Stockholm (SE)
(74) Representative: Trier, Mikkel Roed

(57) **Abstract**

The invention relates to a method for operating a biogas plant, which method comprises the steps of fractionating an organic waste material into a solid and a liquid fraction, leading the solid fraction to a biogas reactor, removing ammonium nitrogen from the liquid fraction, and leading a portion of the liquid fraction, from which nitrogen has been removed, to the biogas reactor.

## Description

The present invention relates to a method for operating a biogas plant.

Biogas has come into focus as an alternative source of energy. Biogas plants takes care of waste products, the disposal of which might otherwise be problematic, and at the same time a valuable fuel is produced, which in many regions of the world comes in as a welcome substitute for depleted or dwindling reserves of readily available fossil fuels.

Within the agricultural sector, biogas plants also already today play a role as central fertilizer distribution and exchange facilities, transferring nitrogen, phosphorus and potassium in degassed manure from pigs, cattle and poultry. However, this distributory function of biogas plants could be further refined and generalized in the years to come such as to offer a significant contribution to the overall management of nutrients on a societal level.

Biogas is produced by the biological breakdown of organic matter, often in the absence of oxygen, which process is known as anaerobic digestion. The main constituents of biogas are methane and carbon dioxide, typically constituting 50-80 % and 20-50 %, respectively, as well as a very few percentages of hydrogen sulphide, hydrogen and ammonia, and trace amounts of carbon monoxide, oxygen and free nitrogen. The combustible part thereof is formed by methane and whatever hydrogen might be present, and normally an over-arching endeavour when operating a biogas plant is to maximize not only the total biogasification but also the proportion of methane among the produced gases. Generally, the methane percentage increases when the content of fats in the starting material to be digested is augmented relative to the contents of carbohydrates.

The process of anaerobic digestion is a multi-stage process performed by different groups of microorganisms, wherein the breakdown products of one group serve as the substrate of the next group. The stages are known as hydrolysis, acidogenesis, acetogenesis, all performed by bacteria, and, finally, methanogenesis, which is done by archaea.

Among these stages, methanogenesis is the limiting and most precarious sub-process of the entire anaerobic digestion. The methano-genic archaea produce methane partly from acetic acid and partly from carbon dioxide and hydrogen, and while on one hand they depend on nutrients including nitrogen for their growth, they are inhibited on the other hand by elevated levels of ammonia.

Ammonia is present in equilibrium with ammonium: NH₄⁺ <-> NH₃ + H⁺, which balance is shifted towards the right at high pH and elevated temperature. This implies that when a biogas plant is operated at a temperature corresponding to so-called thermophilic digestion at 49-57 °C (typically 52 °C), the process is more prone to inhibition by ammonia than when performed in the mode known as mesophilic digestion at 30-38 °C (typically 37 °C). Normally, digestion will be stable at pH 6.5-8, but when exceeding this level, the methanogenesis is easily hampered by ammonia.

Consequently, in order to maximize digestion, and thus production, in a biogas plant, it is important to control pH and temperature during the process, but also the content of ammonium in the feed stock to be digested is crucial.

In this regard it has been attempted to control ammonium via dilution of the feed stock by adding extra water to the reactor, or via raising the carbon to nitrogen (C/N) ratio by supplying additional organic material low in nitrogen to the feed stock in case of foreseeable ammonia overloads, but these measures are often imprecise and may affect biogas production adversely.

In view of the above, a major object of the present invention is to provide an efficient, simple and environmentally friendly procedure for controlling the level of ammonium in biogas plants to enhance their productivity.

To meet this object, a method for operating a biogas plant is provided, which method comprises the steps of fractionating an organic waste material with a content of ammonium nitrogen into a solid fraction and a liquid fraction; leading the solid fraction to a biogas reactor; removing ammonium nitrogen from the liquid fraction by applying said liquid fraction to an organic, synthetic ion exchanger adsorbing, in use, more than 1.2 eq/l, preferably more than 2.0 eq/l; allowing ammonium nitrogen from said liquid fraction to adsorb to said ion exchanger, wherein the concentration of ammonium nitrogen in said liquid fraction exceeds 2 g/l at the time of application of said waste water to said ion exchanger, and wherein the liquid fraction has a content of organic matter of not more than 8 % (w/w) at the time of application of said liquid fraction to the ion exchanger, said organic matter being dissolved or being in particles of a maximum extension of 25 µm; and collecting from said ion exchanger a permeate from which ammonium nitrogen has been extensively removed, and leading at least a portion of said permeate to said biogas reactor. Here and in the remainder of the present text, "biogas reactor" should be taken to possibly designate an array of sequentially connected vessels devoted to the process of biogasification, for instance a mixing tank, a digester, and an end-digester, rather than just a single container.

It has surprisingly been found that by diverting ammonia as specified above from the material to be digested, a markedly higher productivity of biogas plants can be attained, and it is made possible to receive types of organic materials, e.g. sorts of manure such as poultry dung, which would hardly otherwise have been eligible as feed stocks for biogasification in any noteworthy proportion due to their high content of nitrogen. For this purpose, it has surprisingly been found that the use of an organic, synthetic ion exchanger makes it possible to remove ammonium nitrogen at a high flow rate and concentration factor directly from a high-ammonium liquid fraction of organic waste material, and in such a manner that these favourable properties of the ion exchanger persist even when it is repeatedly regenerated and exposed to the liquid to be treated for an extended period of time.

Hereby, a robust, efficient, simple low-energy method for operating a biogas plant is provided, so that ammonia inhibition of the digestion may be avoided. At the same time, the possibility of retrieving ammonium nitrogen in a range of valuable forms is opened up.

To meet the aforementioned object, alternatively a second method for operating a biogas plant is provided, which method comprises the steps of fractionating an organic waste material with a content of ammonium nitrogen into a solid fraction and a liquid fraction; leading the solid fraction to a biogas reactor; removing ammonium nitrogen from the liquid fraction by applying said liquid fraction to an organic, synthetic ion exchanger adsorbing, in use, more than 1.2 eq/l, preferably more than 2.0 eq/l, and allowing ammonium nitrogen from said liquid fraction to adsorb to said ion exchanger, wherein the liquid fraction has a content of organic matter of not more than 8 % (w/w) at the time of application of said liquid fraction to the ion exchanger, said organic matter being in particles of a maximum extension of 25 µm; collecting from said ion exchanger a permeate from which ammonium nitrogen has been extensively removed and leading a portion of said permeate to said biogas reactor; and regenerating the ion exchanger with a solution of NaNO₃ of a molality from 3 mol/kg to full saturation and of a temperature from 5 to 40 °C, and/or with a solution of Na₂CO₃ of a molality from 1 mol/kg to full saturation and of a temperature from 5 to 40 °C , and/or with a solution of NaCl of a molality from 3 mol/kg to full saturation and of a temperature from 5 to 40 °C, and/or with a solution of Na₂SO₄ of a molality from 1 mol/kg to full saturation and of a temperature from 30 to 40 °C, and/or with a solution of K₂CO₃ of a molality from 4 mol/kg to full saturation and of a temperature from 5 to 40 °C, and/or with a solution of K₂HPO₄ of a molality from 4 mol/kg to full saturation and of a temperature from 5 to 40 °C. Optionally, the concentration of ammonium nitrogen in said liquid fraction exceeds 2 g/l at the time of application of said waste water to said ion exchanger.

Surprisingly, the inventors have realized that the organic, synthetic ion exchanger in the present application actually tolerates such very strong regenerant solutions despite express exhortations in the directions for use given by producers of synthetic ion exchangers that the latter only be regenerated with much weaker solutions in order not to destroy the ion exchanger as a result of excessive osmotic shock. The possibility of using strong regenerant solutions is a strongly contributory factor in achieving a high concentration factor. Besides, strong saline solutions effectively inhibit the establishment of most kinds of microbiological cultures in the bed of ion exchanger.

Hereby, a robust, simple method for operating a biogas plant is provided, which offers a very efficient recovery of ammonium nitrogen.

The preferred solvent for the solutions applied for regeneration is water, although other suitable solvents may also come into question. The regenerant solutions of the respective salts may be employed singularly or combined. Each ion of ammonium (NH₄⁺) will exchange with one of the likewise monovalent ions of sodium (Na⁺) or potassium (K⁺), respectively, in the regenerant solutions. In this regard, it is to be understood that any of the listed salts into which enter two atoms of sodium or potassium per molecule will offer for ammonium exchange twice as many molar equivalents/kg as the molecular molality cited for the solution.

In the following, various embodiments of the second method according to the invention will be discussed.

In one embodiment, the ion exchanger is regenerated with a solution of K₂CO₃ having a temperature of 5 °C and a molality of more than 5 mol/kg, more than 6 mol/kg, preferentially 7 mol/kg. Most preferred, the ion exchanger is regenerated with a solution of K₂CO₃ of a molality of 8 mol/kg and a temperature of 20 °C.

The ion exchanger may also be regenerated with a solution of NaNO₃ having a temperature of 5 °C and a molality of more than 6 mol/kg, more than 7 mol/kg, advantageously 8 mol/kg. Further, it may be regenerated with a solution of NaNO₃ having a temperature of 10 °C and a molality of 9 mol/kg, or, most preferred, with a solution of NaNO₃ having a temperature of 20 °C and a molality of 10 mol/kg. The use of NaNO₃ as a regenerant is favourable in that ammonium nitrate results as a product. This is much in demand as a high-nitrogen fertilizer and as an explosive for coal and steel mining, quarrying, and construction works.

Likewise, the ion exchanger may be regenerated with a solution of Na₂CO₃ showing a temperature of 20 °C and a molality of 2 mol/kg, a temperature of 30 °C and a molality of 3 mol/kg, or, preferably, a temperature of 40 °C and a molality of 4.5 mol/kg. Ammonium hydrogen carbonate, which is a fertilizer much in demand in China, may advantageously be prepared by using Na₂CO₃ as a regenerant with ensuing passage of fine bubbles of carbon dioxide through the eluate and cooling thereof.

Regeneration of the ion exchanger can also be performed with a solution of Na₂SO₄ presenting a temperature of 30 °C and a molality of 2.5 mol/kg, or, favourably, presenting a temperature of 32 °C and a molality of 3.5 mol/kg. The resulting product, ammonium sulfate, is in demand as a fertilizer for alkaline soils and is moreover employed in vaccines, as a food additive and for purifying proteins by selective precipitation.

The ion exchanger may also be regenerated with a solution of NaCl of a molality of 6 mol/kg and a temperature of 5 °C, 10 °C, or preferably, 20 °C. In this way a method is provided, by which ammonium nitrogen from organic waste water can be recovered in a form having obvious and versatile applications. Ammonium chloride is suitable for use as a feed supplement for cattle and may be converted to a number of fertilizer products by established methods, but it also finds a great many non-agricultural uses in its own right. It is employed, e.g., in textile printing, plywood glue, hair shampoo, cleaning products, in nutritive media for yeast, as cough medicine, to slow the melting of snow on ski slopes at temperatures above 0 °C and as a flavour additive to liquorice and vodka.

Further, the ion exchanger can be regenerated with a solution of K₂HPO₄ of a molality of 5, 6, 7, or, preferably, 8 mol/kg and a temperature of 20 °C.

Generally, the salts of ammonium (and potassium) produced when regenerating the ion exchanger may be separated from the eluate streaming from the ion exchanger by addition of the regenerant salt at a specified temperature at which the solubility of the regenerant differs from the solubility of the ammonium and potassium salts. If the product salts present the lower solubility, they may be recovered as crystals. If they have the higher solubility, they can be recovered from the solution and the regenerant can be recovered as crystals.

In the following, issues which apply for both methods according to the invention will be discussed.

The fractionation of the organic waste material into a solid fraction and a liquid fraction may be achieved by means of any kind of separating device, such as a decanter or a screw press. In the present context, "organic waste material" should be taken to also encompass material such as energy crops, which have been purposively produced for biogasification. Upon fractionation, the solid fraction will typically have a dry matter content of 20-30 %.

It may be relevant to adjust the pH of the organic waste material prior to its fractionation, depending on the nature and freshness of said material. In agricultural, municipal and industrial waste, a substantial part of the nitrogen is often present as ammonium, much of which may possibly originate from the metabolism of animals. When leaving mammals, a considerable proportion of nitrogen in the metabolic waste products is in the form of urea. Shortly thereafter, however, urea is converted into ammonium and carbon dioxide in a pH-neutral mixture. In the following period, then, carbon dioxide leaves, the pH increases and ammonia will start to evaporate. In order to optimally lend itself to the treatment according to the invention, the organic waste material should have a pH in the range of 6.5 to 8.0. Therefore, if the waste material is not fresh, neutralization, e.g. with carbon dioxide, may be called for. Often, the waste material is less amenable to treatment with mineral acids due to excessive foaming.

The organic, synthetic ion exchanger is a cation exchanger preferably made from a gel resin, such as styrene crosslinked by addition of divinylbenzene at the polymerisation process and with strongly acidic functional groups, but may also be of a macroporous type.

Preferably, the ion exchanger is brought on Na⁺-form or K⁺-form prior to the application of the liquid waste fraction to the ion exchanger. For instance, if it has been pre-loaded with H⁺ ions or is entirely virgin it may be treated with a solution of sodium chloride, sodium nitrate or sodium sulphate. Other easily soluble cations, which in combination with the applied ion exchanger resin are suitable for selective exchange of ammonium ions from the liquid to be treated, may also come into consideration for pre-loading of the ion exchanger. Furthermore, older organic waste water rich in ammonia could be applied to a separate bed of organic, synthetic ion exchanger on H⁺-form as an alternative to neutralization.

According to a preferred embodiment, the step of applying the liquid waste fraction to the ion exchanger and a step of regenerating the ion exchanger are performed by turns in a series comprising more than 10, preferably more than 25, most preferred more than 50 repetitions of said steps, wherein the ion exchanger is not replaced during the duration of such a series. The inventors have unexpectedly found that the ion exchanger stands up to such a treatment without any significant impairment of its performance.

In one embodiment, the concentration of ammonium nitrogen in the liquid waste fraction exceeds 3 g/l, preferentially 4 g/l, preferably 5 g/l. Unexpectedly, the use of a durable ion exchanger with a high exchange capacity, i.e. 1.2 molar equivalents per liter (eq/l), preferably 2.0 molar equivalents per liter, renders possible to favourably treat liquids with high concentrations of ammonium by way of ion exchanging without the need for any pretreatment to reduce the ammonium content of the liquid to be treated, which would otherwise not have been practical and profitable.

The liquid waste fraction may have a content of organic matter of more than 1, more than 2, more than 3, or more than 5 % (w/w) at the time of application of said waste water to the ion exchanger, said organic matter being dissolved or in particles of a maximum extension of 25 µm. Surprisingly, such a considerable content of organic matter is reconcilable with the sustained functioning of the bed of organic, synthetic ion exchanger at a high flow rate and ion exchange capacity, despite the fact that organic, synthetic ion exchangers are manufactured and normally used for treatment in industry and research of liquids, which are substantially devoid of particles and organic matter.

In a specific embodiment, the liquid fraction to be treated comprises liquid manure. The liquid manure may originate from any animal, but most often stems from livestock, e.g. pigs, cows or poultry. Prior to its application to the ion exchanger said manure may be admixed with other kinds of organic waste, such as municipal sewage.

The biogas plant may be installed at a central plant receiving manure-containing waste from several external sources or it may be put up in a farm setting to be associated with a stable, be it a traditional or a loose-housing system, or a pigsty, be it indoors or outdoors. By the latter association the possibility of a predictable and stable supply of fresh manure is assured.

Preferably, the liquid manure results from a fractionation of manure, such as to restrict the occurrence of coarse, solid matter. Optionally, the manure is briefly stored in a reservoir before fractionation. The fractionation may be achieved by means of any kind of separator, optionally a screen shaker separator. The manure may also be separated in a decanter, optionally following treatment in a screw press.

Advantageously, the liquid manure is fractionated and, after shortly residing in one or more buffer tanks, applied to the ion exchanger within a period from 2 days to 5 weeks after the occurrence of the underlying, causative defecation and urination to limit the emission of ammonia and assure that the manure is still relatively fresh and is easy to fractionate. Processing the manure at such an early stage presents the additional advantage that the emission of methane and laughing gas, which are greenhouse gases 21 and 289 times as potent as carbon dioxide, respectively, is extensively limited.

To assure that the liquid waste fraction is treated at a stage, where the predominant part of the nitrogen contained therein is present in the form of ammonium, it should not be left to turn alkaline. In case that a substantial part of the ammonium present has been allowed to convert to ammonia, it will be ineffective to apply the organic waste water to the ion exchanger on Na⁺-form or K⁺-form. Instead, liquid waste fraction rich in ammonia as a result of extended storage should as mentioned earlier be neutralized or applied to a separate bed of organic, synthetic ion exchanger on H⁺-form. On the other hand, fresh liquid fraction, wherein the nitrogen is predominantly present in the form of ammonium, must not be applied to an ion exchanger on H⁺-form, even though this is the default loading of many commercial ion exchangers. Such applications will result in an effervescence of carbon dioxide of explosive character.

Preferentially, the beads of the ion exchanger have a mean particle size of 0.4-1.0 mm, preferably 0.6-0.7 mm, and a uniformity coefficient of 1.2 or less, preferably 1.1 or less. The uniformity coefficient is defined as the relation between the particle size corresponding to the mesh at which 60% of the particles pass a sieve, and the particle size corresponding to the mesh at which 10% of the particles pass a sieve. If the beads are too large, the accessible surface area of the beads and thus the total exchange capacity of the bed of ion exchanger will be insufficient, whereas beads, which are too small, will float atop the liquid to be treated rather than being pervaded by it. Further, a low uniformity coefficient assures that the particles of the organic, synthetic ion exchanger are not packed too tightly and are less prone to clogging, especially when compared to natural ion exchangers. A much higher flow rate is made possible when employing an organic, synthetic ion exchanger. Whereas channeling at a low flow rate, and turbulence and flushing out of minor constituent particles at a high flow rate tend to occur in a bed of natural ion exchanger, the inventors have discovered that these phenomena are much less of a problem with organic, synthetic ion exchangers. Further, in a favourable embodiment, the beads of ion exchanger resin may be unpacked with regular intervals by blowing through compressed air from beneath the bed of ion exchanger.

Part of the above procedures form the subject-matter of the co-pending patent application PCT/DK2011/050260, which is hereby incorporated by reference.

According to a preferred embodiment, a content of dry matter of 8-15% (w/w) is maintained in the biogas reactor. This level represents a suitable balance; as substrate for digestion, a substantial amount of dry matter is desirable, but still the contents of the reactor should be stirrable and liquid should be present to mediate the contact between the substrate and the digesting microorganisms. The desired content of dry matter in the biogas reactor is achieved, partially or exclusively, by supply of said ion exchanger permeate, from which ammonium nitrogen has been extensively removed, to the biogas reactor.

Preferably, the biogas produced in said biogas reactor is upgraded by separation of carbon dioxide from methane, which separation comprises the steps of introducing a stream of said biogas into a bed of a weakly basic ion exchange resin provided with amine groups, at temperature and pressure conditions, under which the carbon dioxide is adsorbed to said resin, and desorbing the adsorbed carbon dioxide from the resin by increasing the temperature and/or lowering the pressure in said bed, wherein content of water in said bed of ion exchange resin during the step of biogas introduction amounts to more than 5, more than 10, more than 20, preferably more than 35 % of the total weight of ion exchange resin and water, and wherein a portion of the carbon dioxide desorbed from said resin is returned to the biogas production process by being led to the organic waste material for pH adjustment thereof prior to the fractionation of said material into said solid fraction and said liquid fraction.

By this partial recycling of internally produced carbon dioxide, pH adjustment is henceforth inherently integrated into the biogas production process in a most beneficial way, obliterating any need for supply of extraneous chemicals for this purpose.

It has surprisingly been found that the presence of a considerable amount of unsorbed water in the bed of ion exchange resin for carbon dioxide adsorption do not inhibit clearing of carbon dioxide from the stream of biogas to be upgraded, but actually results in an overall capacity for carbon dioxide removal, which is far superior to that achieved by conventionally operated temperature/pressure swing adsorption as well as by water scrubbing.

Thus, a robust and effective procedure is provided for upgrading of the gas produced in the biogas plant, which procedure can be performed within a relatively modest space.

The ion exchange resin employed for this purpose preferably is of a macroporous type in order to provide a large surface for adsorption. Its matrix may typically be composed of a polystyrene cross-linked with divinylbenzene. The functional group preferentially is a tertiary amine.

Depending on the intended end-use of the biogas as well as the composition of the organic waste material entering into the biogas plant, it may be relevant to remove hydrogen sulphide from the crude gas stream prior to its introduction into the bed of ion exchange resin. Often-times, this will be required to forestall corrosion of vessels and engines. Moreover, such removal may be desirable for the purpose of recycling of carbon dioxide to said organic waste material for pH adjustment prior to digestion. Methods for hydrogen sulphide removal are well-known within the art.

Preferably, the content of water in the bed of said ion exchange resin during the step of introducing a stream of biogas amounts to 37% or more, 40% or more, preferentially 45% or more, advantageously 50% or more, optionally 55% or more, of the total weight of ion exchange resin and water. It has been found that the total capacity for combined adsorption and absorption of carbon dioxide from the stream of biogas as well as the adsorption rate culminate close to a water content of 50%.

Advantageously, the content of water in said bed of ion exchange resin during the step of introducing a stream of biogas amounts to less than 80%, less than 75%, less than 72%, preferably less than 70%, optionally less than 67%, less than 65% or less than 60% of the total weight of ion exchange resin and water. At water shares exceeding 80%, the mixture of ion exchange resin and water takes on the appearance of a diffluent slurry with a layer of resin beads floating atop, and the favourable synergistic effect of CO₂-absorption in the water and CO₂-adsorption to the ion exchange resin is no longer present.

Optionally, a flow of water counter to the stream of biogas is provided during the step of combined adsorption and absorption, when carbon dioxide is introduced to the bed of ion exchange resin. The flow of water is introduced as a spray above the bed of ion exchange resin, whereas the stream of biogas is concomitantly introduced to the bottom of said bed. The produced counter-flow of water emulates the principle of a water-scrubber and has a certain effect in absorbing and driving downwards residual carbon dioxide that passes through the wet bed of ion exchanger and rises above its reach.

Preferably, the temperature of the bed of ion exchange resin is increased in the step of desorption by injecting warm water directly into said bed. Typically, the bed of ion exchange resin will be warmed to a temperature of 20-100 °C, preferably 40-70 °C, at atmospheric pressure.

Preferentially, the temperature of the bed of ion exchange resin is lowered antecedently to the adsorption/absorption step of introducing a stream of biogas step by injecting cold water directly into said bed. Usually, the ion exchanger bed will be cooled to a temperature of -20-20 °C, often 0-10 °C, for choice approximately 5 °C, at a pressure of 2-3 bar (1-2 bar above atmospheric pressure).

Moreover, the cooling and heating of the bed of ion exchange resin may be assisted by one or more heat exchangers in the form of a mantle encircling the bed of ion exchange resin or elements protruding into said bed.

The bed of ion exchange resin may be supplemented by additional similar beds arranged in a serial array of two or more vessels, between which pressure equalization is applied, so that a gas pressure released from one bed is subsequently utilized in one or more other beds.

Part of the above procedures for carbon dioxide removal form the subject-matter of the co-pending patent application DK 201100994, which is hereby incorporated by reference.

According to a preferred embodiment, a degassed substance arising from digestion of said organic waste material in said biogas reactor is led from said reactor and segregated in a solid part and a liquid part, for instance in a screw press, whereupon ammonium nitrogen is removed from said liquid part. Preferably, said removal of ammonium is effected by applying said liquid part to an ion exchanger, advantageously an organic, synthetic ion exchanger adsorbing, in use, more than 1.2 eq/l, preferably more than 2.0 eq/l; allowing ammonium nitrogen from said liquid part to adsorb to said ion exchanger, wherein the liquid part has a content of organic matter of not more than 8 % (w/w) at the time of application of said liquid part to the ion exchanger, said organic matter being in particles of a maximum extension of 25 µm; and, optionally, regenerating the ion exchanger with a solution of NaNO₃ of a molality from 3 mol/kg to full saturation and of a temperature from 5 to 40 °C, and/or with a solution of Na₂CO₃ of a molality from 1 mol/kg to full saturation and of a temperature from 5 to 40 °C , and/or with a solution of NaCl of a molality from 3 mol/kg to full saturation and of a temperature from 5 to 40 °C, and/or with a solution of Na₂SO₄ of a molality from 1 mol/kg to full saturation and of a temperature from 30 to 40 °C, and/or with a solution of K₂CO₃ of a molality from 4 mol/kg to full saturation and of a temperature from 5 to 40 °C, and/or with a solution of K₂HPO₄ of a molality from 4 mol/kg to full saturation and of a temperature from 5 to 40 °C.

From the eluate flowing from the ion exchanger as a result of said regeneration, fertilizers rich in nitrogen and potassium may be produced.

During the process of digestion, the ratio between nitrogen available as ammonium and nitrogen incorporated in organic matter increases, and as ammonium is released, the risk of evaporation of ammonia also rises. It may therefore be relevant to treat not only the liquid part of the final degassed substance with the above procedure for removal of ammonium but also to clear the liquid part of intermediate digestion products from ammonium in the same manner in order to avoid insidious ammonia inhibition of the methanogenesis. At all events, a permeate or a portion thereof from said ion exchanger may be recycled to the biogas reactor for adjustment of its relative content of dry matter.

According to a preferred embodiment, said degassed substance, and possibly also said intermediate digestion products, are adjusted with regard to their pH with carbon dioxide provided by the gas separation according to the procedure described in the above, prior to the removal of ammonium nitrogen from their liquid parts. The supply of carbon dioxide serves to shift the equilibrium from ammonia towards ammonium.

In a preferred embodiment, the solid part of the degassed substance is formed to pellets, which are dried. The pellets can either be combusted for energy or pyrolized with an eye to production of a fertilizer rich in phosphorus.

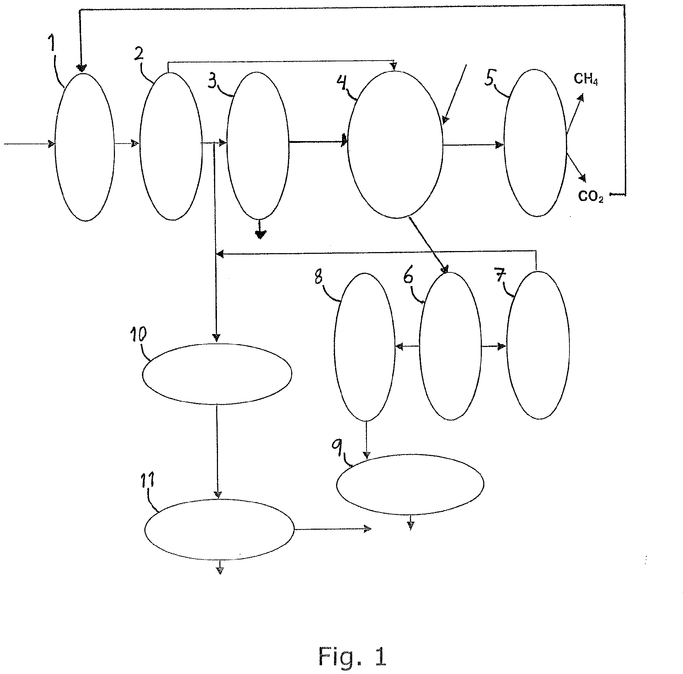

In the following, a preferred embodiment of the invention will be illustrated by reference to the non-limiting figure. The figure shows a schematic view of an embodiment of plant for carrying out the methods according to the invention.

Referring now to the figure, the main features of the illustrated plant are referenced by numbers as follows:
1 is a vessel or a site for pH-adjustment of incoming organic waste material; 2 is a separation device for fractionating the organic waste material into a solid fraction and a liquid fraction; 3 is a device comprising an ion exchanger for removing ammonium nitrogen from said liquid fraction; 4 is a biogas reactor array comprising a sequence of vessels; 5 is a device for upgrading the produced biogas by separation of carbon dioxide and methane; 6 is a separation device for segregating a degassed substance into a solid part and a liquid part; 7 is a device comprising an ion exchanger for removing ammonium nitrogen from said liquid part; 8 is a site for forming and drying pellets from said solid part; 9 is a site for burning or pyrolysis of pellets; 10 is a site for pretreatment of liquids with a content of phosphorus; and 11 is a device for recovery of phosphorus.

A description of a preferred embodiment of the invention as carried out in the plant of the figure will now be given.

Liquid manure is received at the site 1 together with other organic waste materials intended for biogasification. When arriving, the manure may present itself as a relatively fresh, thin slurry, wherein a pronounced majority of nitrogen is present as ammonium, pH is neutral and the content of carbonic acid is high. If, on the other hand, the manure or the other waste materials have been stored for a longer period and have turned ammoniacal, pH is adjusted by supplying carbon dioxide from the device 5 for upgrading biogas. After residing at the site 1 for no more than a few days, portions of the mixture of organic waste materials are conveyed with regular intervals to the separation device 2 in form of a decanter centrifuge, to be separated into two fractions. One fraction is a solid fibre fraction and the other fraction is a liquid fraction having substantially no particles larger than 25 µm. The liquid fraction is stored in a buffer tank (not shown) for only long enough to ensure that substantially all urea from the manure is converted to ammonium and carbon dioxide. The solid fraction is transported to a mixing tank of the biogas reactor array 4, which further comprises a digester and an end-digester.

From said buffer tank, the liquid fraction containing ammonium nitrogen in a concentration of 4 g/l and 2% (w/w) of organic matter at this stage, is pumped to the device 3 for ammonium removal. This device encompasses two parallelly arranged containers with a bed of organic, synthetic ion exchanger within them. In case that large quantities of liquid waste fraction were to be treated, further containers connected in parallel might have been present. The ion exchanger is made of a gel resin on Na⁺-form, having as its matrix styrene crosslinked by addition of divinylbenzene and having as functional group sulfonic acid. The total exchange capacity of the ion exchanger amounts to about 2 molar equivalents per litre, and the average bead size is about 0.65 mm, showing a uniformity coefficient of about 1.1. A volume of approximately 1.6 m³ of ion exchanger is present in each container, and the inner cross-sectional area of each container at the top level of the bed of ion exchanger is around 1.8 m².

The liquid to be treated is pumped to the top of each container such as to percolate through the bed of synthetic, organic ion exchanger by the force of gravity at a flow rate of 3-10 cm/min. The operation proceeds at atmospheric pressure; however, at regular intervals the bed of ion exchanger is blown through by compressed air at a maximum of 2.0 bars from the bottom of the container in order to maintain a porous, homogenous overall structure of the bed.

Part of the permeate is led to the mixing tank of the biogas reactor array 4 for admixing with the solid fibre fraction with a view to maintaining a dry matter content in said array of approximately 12% (w/w). The remaining part of the permeate is adjusted to a prescribed water quality in an ultrafiltration unit and a reverse osmosis unit (not shown) to finally arrive in a buffer tank (not shown either), from which it is discarded or put to a suitable use according to local demands. Otherwise, its use as a dilute fertilizer could have been desirable.

In the event that the biogas plant had been associated with a farm, the permeate could advantageously have been put to use in the continuous or intermittent flushing of manure from beneath the floor of a stable or pigsty with an eye to restricting the conversion of nitrogen in the manure from ammonium into ammonia. Preferably, the flushed manure including the permeate used for flushing would form the basis of the liquid waste fraction to be applied to the ion exchanger. Suitably, the flow of liquid manure, provided by said flushing using permeate from the ion exchanger, would have been timed such as to ascertain the conversion of urea contained in the manure into ammonium and carbon dioxide, while still restricting the conversion of ammonium into ammonia.

In this way, the permeate might have been turned to account in a most propitious way, as the flow of manure would now be intimately linked to the process for removal of ammonium nitrogen. Consequently, the manure would enter into a regular flow and would still be fresh when applied to the ion exchanger. Hereby, the emission of ammonia to the air of the stable or pigsty might be reduced by as much as 60% or more, and the ratio of ammonium to ammonia in the liquid manure to be treated would be sufficiently high to assure that a substantial part of the nitrogen present might be scavenged as ammonium ions in the ion exchanger. Conversely, if manure stored in a traditional way for a longer period in a manure tank or lagoon is to be cleansed from nitrogen by use of an ion exchanger, ammonia will be more prevalent and a more extensive supply of carbon dioxide from the device 5 will be required for neutralization. Alternatively, a step of separate treatment in a bed of H⁺-loaded ion exchanger to be regenerated with a solution of phosphoric acid or sulphuric acid should have been included, if a similar effectiveness was to be attained.

Moreover, by recycling permeate instead of flushing with water, substantial savings might be gained and furthermore the flushing with permeate would not add to the overall volume of manure, as the fluid used in flushing itself originates from manure.

In the present embodiment, the supply of liquid waste fraction to a bed of ion exchanger is interrupted when ammonium in a pre-specified concentration as determined by online measurements begins to leak from its bottom. Regeneration of the ammonium-saturated container is started while a fresh container is switched in to replace it in the ion exchange treatment of liquid waste fraction. In this way a continuous operation of the plant is effected.

Before regeneration, however, the respective bed of ion exchanger is flushed with one bed volume of water such as to rinse out particulate matter and organic material from the ion exchanger.

The regeneration is performed with NaNO₃ in a concentration of about 8 mol/kg, corresponding to almost complete saline saturation, which is introduced to the bottom of the ion exchanger container from the vessel 12. At such a concentration, bacteria and fungi that might have been present in the bed of the ion exchanger are killed off to an extent that any preceding pasteurization can be omitted. The applied ions of sodium act such as to replace adsorbed ions of potassium and subsequently ions of ammonium as well as some amino acids from the ion exchanger. The supply of saline solution is upheld until a pre-specified low level of ammonium is reached in the eluate leaving the bed of ion exchanger, whereupon the latter is rinsed again with water to clear it from sodium nitrate. Then the ion exchanger is ready again for treatment of the liquid waste fraction.

Said rinsing water and the eluate is led to a buffer tank (not shown) as a solution of NH₄NO₃ and KNO₃. Subsequently, it is brought to a mixing tank (not shown), wherein a high-grade fertilizer is produced by adjusting the proportions in said solution of the most prevalent macronutrients. Suitable formulations of nitrogen, phosphorus and potassium are supplied from a number of vessels (not shown), and other nutrients might have been added as well.

When operating according to the procedure outlined above, a very high proportion of the ammonium ions contained in six bed volumes of liquid waste fraction comprising liquid manure may be adsorbed to a single bed of organic, synthetic ion exchanger and be released into one bed volume or less of regenerant solution. In this way a high concentration factor may be obtained and a large proportion of ammonium nitrogen can be diverted from the digestion process.

Within the biogas reactor array 4, the digester is continuously fed with feed stock from the mixing tank, i.e. solid fibre fraction mixed with N-depleted liquid fraction, and a low level of ammonium in the feed stock is aimed at. Already an ammonium content of 200-500 ppm may retard fermentation and at 5000 ppm ammonium, biogas production may well terminate, especially at higher temperatures.

To the mixing tank or the digester are added supplementary materials for co-fermentation, notably plant residues such as straw and energy crops such as clover, hemp and napier grass. The residence time of the waste materials in the digester depends on the actual assortment of said materials and the temperature of the digestion process. Due to the fact that the methanogenic archaea thanks to the procedures of the present invention are relieved of a considerable ammonium/ammonia stress, it is possible to run the process as a thermophilic digestion at 52 °C with a residence time of 20 days. The last phase of the digestion takes place in the end-digester, to which the digestate is transferred when approximately 80 % of its biogas potential has been exhausted.

The produced biogas is led to the device 5 for upgrading biogas. First, it is passed through a column of activated carbon impregnated with potassium iodide to extensively remove hydrogen sulphide from the biogas.

A vessel (not shown) contained in said device 5 is prepared for adsorption by cooling ion exchanger contained therein to 5 °C by injection of cold water. When the resin has been cooled, the vessel is drained to the point, at which the content of water in the bed of ion exchange resin amounts to approximately 50 % of the total weight of ion exchange resin and water.

After having been cleansed from hydrogen sulphide, the biogas is introduced at 5 °C and 100 % relative humidity to the bottom of the vessel at a pressure of 2 bar (1 bar above atmospheric pressure) and a flow rate of 15 bed volumes per hour. The biogas in the present case is mainly composed of carbon dioxide and methane in a ratio of 40/60. Carbon dioxide is absorbed in the water and adsorbed to the ion exchange resin, and methane of a high purity is led out of a valve in the top of the vessel. The pressure is kept constant at 1.5-2 bar.

It is assumed that the observed propitious synergy of carbon dioxide absorption and adsorption owes itself to the fact that the water acts as a mediator between carbon dioxide in its gas phase and the solid adsorbent resin.

The content of carbon dioxide in the methane leaving the valve in the top of the vessel is continuously monitored. When the bed of ion exchange resin and water is saturated at 25-30 bed volumes, the supply of biogas is stopped and any methane with a content of carbon dioxide exceeding the stipulated limit value is led to a storage vessel (not shown) for subsequent repurification.

Now, the vessel containing the ion exchanger is prepared for desorption by draining off the water contained therein. The pressure is slowly regulated to atmospheric pressure and the vessel is heated from 5 °C to 70 °C by injection of water with a temperature of 70-80 °C. During the heating process, adsorbed CO₂ is released from the bed of ion exchanger, and carbon dioxide of a high purity is collected from the outlet in the top of the vessel.

When desorption is substantially complete, the bed of ion exchange resin is ready for being prepared for adsorption as initially described.

The resulting upgraded biogas in the form of methane of a purity of 95-98 % may be subjected to drying in a gas drying system and possibly compression as required by the end users, while the separated carbon dioxide may be utilized in various enterprises, e.g. greenhouses. As described earlier, it also has an important function in neutralizing waste material received in the biogas plant at the site 1.

From the end-digester, degassed substance is led to the separation device 6, which is a screw press for segregating said substance into a solid fibre part and a liquid part. The liquid part is neutralized as required by bubbling through carbon dioxide produced in the biogas upgrading device 5, before being subjected to ammonium removal by ion exchange in the device 7.

The solid part, containing substantially all particles showing a maximum extension of more than 20 µm, is dried from a dry matter content of about 30% to a dry matter content of about 80% in a stream of hot exhaust from a generator driven by the produced biogas. The dried fibre substance is also pressed to pellets at the site 8, from where they are transported to the site 9, where they are burned for heat energy or subjected to pyrolysis with a view to subsequent extraction of phosphorus.

Finally, permeate arising from the devices 3 and 7 for ammonium removal may be led to the pretreatment site 10, at which site they are subjected to pretreatment steps such as flotation, filtration and biological treatment, before they are directed to the device 11 for recovery of phosphate with anion exchangers.

### Examples

### Example 1: Demonstration of scope for enhanced capacity utilization of a biogas plant

During a period of 7 weeks, the invention was tested at Bånlev Biogas Plant, Jutland, Denmark, by the research institution Agrotech.

Raw manure was separated in a decanter centrifuge (GEA West-falia) into a liquid fraction and a solid fibre fraction. In the liquid fraction, the concentration of ammonium nitrogen was reduced from 2.7 g/kg to 0.2 g/kg (down by 93%) following adsorption to the synthetic, organic ion exchanger. For potassium, the reduction was 2.0 g/kg to 0.2 g/kg (90%). On the basis of this results and a range of further mass balance measurements, a model calculation was set up by Agrotech to illustrate the possibility of using the first method according to the invention for enhancing the capacity utilization at a manure-based biogas plant.

Two modes of operation were compared. In the standard mode, 450,000 tons of raw manure is received per annum and fed to the biogas reactor.

In the alternative mode, 450,000 tons of raw manure is fractionated in a decanter centrifuge. The solid fibre fraction is fed to the biogas reactor together with an amount of liquid low in nitrogen resulting from treatment of the liquid fraction according to the invention. In this regard, a dry matter (DM) content in the biogas reactor of 11.8% is aimed at.

| | Raw manure processing | Fractionation + N removal |
|---|---|---|
| Raw manure, 5% DM (tons/year) | 450,000 | 0 |
| Manure fibre, 28% DM (tons/year) | 0 | 54,000 |
| N-depleted liquid, 2% DM, (tons/year) | 0 | 89,000 |
| Total input, fresh weight (tons/year) | 450,000 | 143,000 |
| Dry matter (tons/year) | 22,500 | 16,900 |
| % DM in biogas reactor input | 5.0 | 11.8 |
| Ammonium-N in reactor (g/kg) | 3.74 | 3.15 |

In this example, the elevated concentration of dry matter brings about a reduction in the input of weight equivalents of fresh organic waste material to the biogas reactor from 450,000 tons/year to 143,000 tons/year. Given an unchanged residence time in the reactor, capacity is released corresponding to an extra 307,000 tons/year of weight equivalents of fresh organic waste material and an ensuing augmented methane production. Accordingly, an existing biogas plant with a capacity of 450,000 tons of raw manure per annum can be optimized by virtue of the invention to handle manure dry matter corresponding to approximately 1,400,000 tons of raw manure per annum. Although it will be noted that a certain amount of dry matter now circumvents the biogas reactor and therefore is not available for digestion, a considerable rise in production is envisaged.

Generally, the content of ammonium-N in the biogas reactor should be less than 5 g/kg for mesophilic operation and less than 4 g/kg for thermophilic operation. Consequently, the conditions for thermophilic, i.e. highly productive operation is fulfilled when employing the procedures of the invention.

### Example 2: Test of different types of organic, synthetic ion exchangers

Two organic, synthetic cation exchangers being of the gel resin type and the macroporous type, respectively, were brought on Na-form and compared with regard to their capacity for ammonium retention.

| Applied bed volumes of solution of NH₄⁺-N (5 g/l) | Dowex G-26 gel resin cation exchanger with strongly acidic functional groups, Na-form Ammonium retention % | Dowex M-31 macroporous cation ex-changer with strongly acidic functional groups, Na-form Ammonium retention % |
|---|---|---|
| 0.0 | 100.0 | 100.0 |
| 0.8 | 100.0 | 100.0 |
| 1.0 | 100.0 | 100.0 |
| 1.2 | 100.0 | 100.0 |
| 1.4 | 100.0 | 100.0 |
| 1.6 | 100.0 | 100.0 |
| 1.8 | 100.0 | 100.0 |
| 2.0 | 100.0 | 100.0 |
| 2.2 | 100.0 | 100.0 |
| 2.4 | 100.0 | 100.0 |
| 3.2 | 100.0 | 100.0 |
| 4.0 | 100.0 | 95.3 |
| 4.8 | 98.0 | 92.2 |
| 5.6 | 89.0 | 37.8 |
| 6.4 | 69.0 | 22.2 |
| 7.2 | 46.0 | 10.0 |
| 8.0 | 7.0 | 0.0 |

Even though the ion exchanger of the gel resin type showed the best purification properties, the macroporous ion exchanger was also found to be fully applicable for the purpose according to the invention.

### Example 3: Separation efficiency of selected nutrients

A full-scale plant for carrying out nitrogen removal from organic waste material according to the invention was set up at Wageningen University, Swine Research Centre Sterksel, Netherlands. Incoming pig manure one week old was separated into a solid and a liquid fraction with the aid of a decanter. The liquid fraction was shortly stored in a buffer tank, from which it was pumped onto an organic, synthetic ion exchanger.

The ion exchanger was constituted by beads of a gel resin on Na⁺-form, having as their matrix styrene crosslinked by addition of divinylbenzene and presenting as functional group sulfonic acid. The total exchange capacity of the ion exchanger amounted to approximately 2 molar equivalents per litre, while the average bead size was about 0.65 mm. The uniformity coefficient of the bulk of ion exchanger beads was about 1.1. A volume of approximately 1.6 m³ of ion exchanger was present in each container in a row of containers, and the inner cross-sectional area of each container at the top level of the bed of ion exchanger was approximately 1.8 m².

The liquid to be treated was pumped to the top of each container such as to percolate through the beds of synthetic, organic ion exchanger by the force of gravity at a flow rate of approximately 7 cm/min. Upon saturation of the respective beds of ion exchanger, as defined by a pre-specified ammonium leakage threshold, they were regenerated with a solution of Na-NO₃ at a concentration of about 4 mol/kg, yielding an eluate with nutrients, which had been adsorbed by the ion exchanger. The regeneration was continued until a pre-specified low level of ammonium in the eluate was reached.

The separation efficiency is a measure of the proportion of the mass input per nutrient that ends up in the eluate after being treated according to the above procedure. The separation efficiency was calculated by dividing the mass of nutrient in the eluate with the mass input of the nutrient.

Test 1: A total of 7304 kg of liquid fraction with an organic matter content of 1.6% (w/w) and an ammonium nitrogen content of 4.3 g/l was treated.

| Nutrient | Total N | Total K | NH₄-N |
|---|---|---|---|
| Separation efficiency (%) | 64 | 97 | 87 |

Test 2: A total of 6476 kg of liquid fraction presenting an organic matter content of 1.0% (w/w) and an ammonium nitrogen content of 1.9 g/l was treated.

| Nutrient | Total N | Total K | NH₄-N |
|---|---|---|---|
| Separation efficiency (%) | 60 | 93 | 89 |

As appears, very high separation efficiencies for potassium as well as ammonium nitrogen were found. However, inasmuch as the operations of saturation and regeneration of the ion exchanger were performed with reference to pre-specified ammonium thresholds as mentioned in the above, the separation efficiencies may well be further augmented to a value close to 100% if desired by adjusting said thresholds.

### Example 4: Resistance of ion exchanger against osmotic shocks

A test was made to find out how repeated osmotic shocks would affect the organic, synthetic ion exchanger. Solutions of 4 mol/kg NaNO₃ and 1% (w/w) of NH₄Cl were applied by turns every 10 minutes to a bed of organic, synthetic ion exchanger. 50 cycles were run, meaning that the ion exchanger was subjected to 100 shifts of solution, which may each be considered an osmotic shock. Subsequently, a random sample of ion exchanger beads was sent to the manufacturer for analysis. It was found that approximately 5% of the beads were cracked. However, the original content of un-cracked beads in the virgin ion exchanger was only guaranteed to a minimum proportion of 95%. Accordingly, no significant deteriorating effect of the osmotic shock treatment was found.

### Example 5: Persistence of capacity and flow

Even after 12 months of continuously full scale processing of liquid manure in a plant operating according to the method of the invention and without any replacement of ion exchanger material from the plant, no problems related to lowered ion exchange capacity, decreased flow rate or bacterial growth turned up.

### Example 6: Persistence of ammonium separation efficiency for different types of ion exchangers during multiple cycles of adsorption and regeneration

Two organic, synthetic cation exchangers of the gel resin type and the macroporous type, respectively, were brought on Na⁺-form and compared with regard to their capacity for sustained ammonium retention at a flow rate of 3 bed volumes per hour. Following adsorption of ammonium to the ion exchanger, the latter was regenerated every time with a solution of NaNO₃ at a molality of 8 mol/kg water and a temperature of 20 °C. A total of 10 runs were performed for the two tested ion exchangers.

| Applied bed volumes of solution of NH₄⁺-N (5 g/l) filtered to 25 µm | Dowex G-26 gel resin cation exchanger with strongly acidic functional groups, Na-form Ammonium retention (%) Run no. 10 | Dowex M-31 macroporous cation exchanger with strongly acidic functional groups, Na-form Ammonium retention (%) Run no. 1 | Dowex M-31 Run no. 5 | Dowex M-31 Run no. 10 |
|---|---|---|---|---|
| 0.5 | 100 | 100 | 100 | 100 |
| 1.0 | 100 | 99 | 99 | 100 |
| 1.5 | 100 | 95 | 99 | 97 |
| 2.0 | 100 | 91 | 92 | 91 |
| 2.5 | 100 | 87 | 86 | 86 |
| 3.0 | 98 | 84 | 84 | 81 |
| 3.5 | 95 | 65 | 63 | 65 |

Albeit the best sustained ammonium purification properties were found for the ion exchanger of the gel resin type, the macroporous ion exchanger was also found to keep up its satisfactory retention level.

## Claims

1. A method for operating a biogas plant, comprising the steps of
(i) fractionating an organic waste material with a content of ammonium nitrogen into a solid fraction and a liquid fraction;
(ii) leading the solid fraction to a biogas reactor;
(iii) removing ammonium nitrogen from the liquid fraction by
a) applying said liquid fraction to an organic, synthetic ion exchanger adsorbing, in use, more than 1.2 eq/l, preferably more than 2.0 eq/l;
b) allowing ammonium nitrogen from said liquid fraction to
adsorb to said ion exchanger,
wherein the concentration of ammonium nitrogen in said liquid fraction exceeds 2 g/l at the time of application of said waste water to said ion exchanger, and wherein the liquid fraction has a content of organic matter of not more than 8 % (w/w) at the time of application of said liquid fraction to the ion exchanger, said organic matter being dissolved or being in particles of a maximum extension of 25 µm; and
(iv) collecting from said ion exchanger a permeate from which ammonium nitrogen has been extensively removed and leading a portion of said permeate to said biogas reactor.

2. A method for operating a biogas plant, comprising the steps of
(i) fractionating an organic waste material with a content of ammonium nitrogen into a solid fraction and a liquid fraction;
(ii) leading the solid fraction to a biogas reactor;
(iii) removing ammonium nitrogen from the liquid fraction by
a) applying said liquid fraction to an organic, synthetic ion exchanger adsorbing, in use, more than 1.2 eq/l, preferably more than 2.0 eq/l;
b) allowing ammonium nitrogen from said liquid fraction to
adsorb to said ion exchanger,
wherein the liquid fraction has a content of organic matter of not more than 8 % (w/w) at the time of application of said liquid fraction to the ion exchanger, said organic matter being in particles of a maximum extension of 25 µm;
(iv) collecting from said ion exchanger a permeate from which ammonium nitrogen has been extensively removed and leading a portion of said permeate to said biogas reactor; and
(v) regenerating the ion exchanger with a solution of NaNO₃ of a molality from 3 mol/kg to full saturation and of a temperature from 5 to 40 °C, and/or with a solution of Na₂CO₃ of a molality from 1 mol/kg to full saturation and of a temperature from 5 to 40 °C , and/or with a solution of NaCl of a molality from 3 mol/kg to full saturation and of a temperature from 5 to 40 °C, and/or with a solution of Na₂SO₄ of a molality from 1 mol/kg to full saturation and of a temperature from 30 to 40 °C, and/or with a solution of K₂CO₃ of a molality from 4 mol/kg to full saturation and of a temperature from 5 to 40 °C, and/or with a solution of K₂HPO₄ of a molality from 4 mol/kg to full saturation and of a temperature from 5 to 40 °C.

3. The method according to any one of the preceding claims, wherein a content of dry matter of 8-15% (w/w) is maintained in the biogas reactor.

4. The method according to any one of the preceding claims, wherein biogas produced in said biogas reactor is upgraded by separation of carbon dioxide from methane, which separation comprises the steps of
i) introducing a stream of said biogas into a bed of a weakly basic ion exchange resin provided with amine groups, at temperature and pressure conditions, under which the carbon dioxide is adsorbed to said resin, and
ii) desorbing the adsorbed carbon dioxide from the resin by increasing the temperature and/or lowering the pressure in said bed,
wherein the content of water in said bed of ion exchange resin during step i) amounts to more than 35 % of the total weight of ion exchange resin and water, and wherein a portion of the carbon dioxide desorbed in step ii) is led to said organic waste material for pH adjustment thereof prior to the fractionation of said material into said solid fraction and said liquid fraction.

5. The method according to any one of the preceding claims, wherein a degassed substance arising from digestion of said organic waste material in said biogas reactor is led from said reactor and segregated in a solid part and a liquid part, whereupon ammonium nitrogen is removed from said liquid part by
α ) applying said liquid part to an organic, synthetic ion exchanger adsorbing, in use, more than 1.2 eq/l, preferably more than 2.0 eq/l;
β ) allowing ammonium nitrogen from said liquid part to adsorb to said ion exchanger, wherein the liquid part has a content of organic matter of not more than 8 % (w/w) at the time of application of said liquid part to the ion exchanger, said organic matter being in particles of a maximum extension of 25 µm; and
γ ) regenerating the ion exchanger with a solution of NaNO₃ of a molality from 3 mol/kg to full saturation and of a temperature from 5 to 40 °C, and/or with a solution of Na₂CO₃ of a molality from 1 mol/kg to full saturation and of a temperature from 5 to 40 °C , and/or with a solution of NaCl of a molality from 3 mol/kg to full saturation and of a temperature from 5 to 40 °C, and/or with a solution of Na₂SO₄ of a molality from 1 mol/kg to full saturation and of a temperature from 30 to 40 °C, and/or with a solution of K₂CO₃ of a molality from 4 mol/kg to full saturation and of a temperature from 5 to 40 °C, and/or with a solution of K₂HPO₄ of a molality from 4 mol/kg to full saturation and of a temperature from 5 to 40 °C.

6. The method according to claim 5, wherein said degassed substance is adjusted in pH with carbon dioxide provided by the gas separation according to claim 4 prior to the removal of ammonium nitrogen from its liquid part.

7. The method according to claims 5 or 6, wherein said solid part of said degassed substance is formed to pellets, which are dried.
